# EUROPEAN PATENT SPECIFICATION

(11) **EP 2 941 212 B1**
(45) Date of publication and mention of the grant of the patent: **24.11.2021**
(21) Application number: 13864447.1
(22) Date of filing: 18.12.2013
(51) Int. Cl.: A61B 17/435, A61B 17/00

(54) **APPARATUS FOR CONTROLLING IN VITRO FERTILIZATION**
VORRICHTUNG ZUR KONTROLLE VON IN-VITRO-BEFRUCHTUNG
APPAREIL DE COMMANDE DE FÉCONDATION IN VITRO

(30) Priority: 18.12.2012 US 201261738735 P; 18.09.2013 US 201361879669 P
(43) Date of publication of application: 11.11.2015
(73) Proprietor: Drummond Scientific Company, Broomall, PA 19008 (US)
(72) Inventor: DRUMMOND, Michael, E., Malvern, PA 19355 (US); DITROLIO, Nicholas, M., Havertown, PA 19083 (US)
(74) Representative: Manitz Finsterwald Patent- und Rechtsanwaltspartnerschaft mbB
(86) International application number: PCT/US2013/076315
(87) International publication number: WO 2014/100294

(56) References cited:
- WO-A1-2009/018521
- US-A- 4 533 345
- US-A- 5 147 315
- US-A- 5 827 174
- US-A1- 2003 199 085
- US-A1- 2004 122 286
- US-A1- 2006 089 608
- None

## Description

### Field of the Invention

The present invention relates, in general, to novel devices for performing *in vitro* fertilization ("IVF"). More particularly, the invention relates to devices for extracting zygotes from a culture or growth medium, and implanting the zygotes on the uterine wall of a patient.

### Background of the Invention

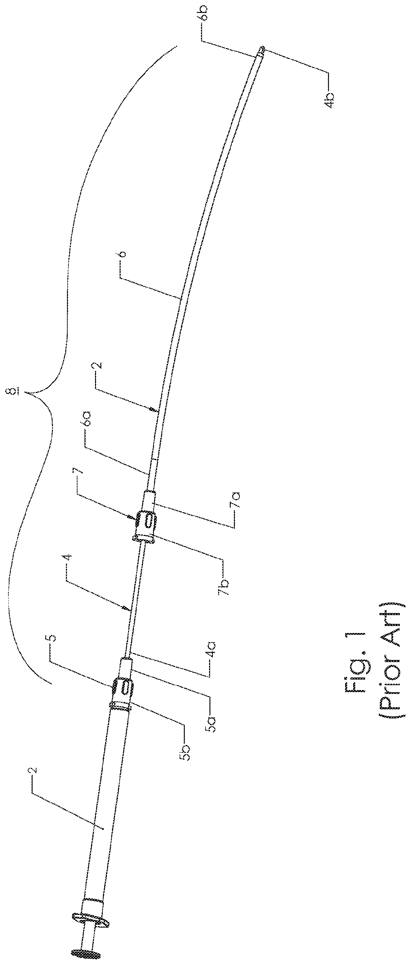

Prior art methods and devices for performing IVF are cumbersome. For example, it is known to perform IVF using a sheathed catheter assembly 8 having a syringe 2 connected to the proximal end of the proximal Luer fitting 5 of the inner catheter 4, such as shown in Fig. 1. To extract a zygote from the growth medium, the catheter 4 is initially primed with growth medium by immersing the distal tip 4b in the growth medium and retracting the syringe to aspirate a small column of growth medium into the catheter. The first zygote is then extracted from the growth medium by locating the distal catheter tip proximate the zygote and retracting the syringe to aspirate the zygote and surrounding growth medium into the inner catheter 4. The steps of priming the catheter 4 and extracting zygotes is repeated several times until a predetermined numbered of zygotes are serially collected in the distal portion of the catheter and surrounded on both sides by a small column of growth medium. US 2003/0199085 A1 disclsoes a method for delivery, a catheter extension, connection and arresting valves and monitoring and pressure discharge systems for use in the field of cell transplantation, regeneration therapy or implantation. Described herein is an apparatus for performing *in vitro* fertilization including a control unit having an external port and means for creating positive and negative fluid pressure at the port, as well as a flexible extension hose that can be connected to the port and is adapted to connect to a Luer fitting of a Luer catheter assembly. WO 2009/018521 A1 discloses an apparatus for separating and storing human reproductive material. The apparatus includes an elongate cryostraw having a first open end and second heat-sealable open end, a channel extending between said ends, and an internal, calibrated volume. A displacement bulb is connected to the first end of the cryostraw for admitting and emitting the material into said cryostraw. The bulb has a total volume, an internal cavity with a compressible end volume, a first seal with the first end of said cryostraw; and, means for limiting the intake volume displacement produced in said cryostraw to an amount less than the calibrated volume no matter how far the bulb is squeezed. U.S. patent no. 5,827,174 B1 discloses a closed sterile in-vitro fertilization system to provide incubation for an oocyte. The system comprises a pouch having a flexible front and a flexible back wall joined at their outer peripheries. The pouch is divided into a first and a second segregated chamber. An entry port is arranged in each of the chambers through the wall of the pouch. A vacuum means is arranged in the first chamber, to permit direct aspiration of an oocyte from an ovary into that chamber, and an entry conduit is arranged between the first and the second chambers, to permit an oocyte to be transferred therebetween.

To implant the zygotes according to one known technique, the outer sheath 6 of the catheter assembly 8 is slid axially forwardly over the distal portion of the inner catheter 4. The distal end of the sheathed catheter assembly end is then guided through the cervix and into the uterus. Once proximate the uterine wall, the inner catheter 4 is extended from the sheath until the distal, open end 4b is immediately adjacent the uterine wall. Next, the zygotes are sequentially implanted by depressing the syringe 2 and expelling the zygotes and surrounding growth medium on to the uterine wall.

To implant the zygotes according to another known technique, the outer sheath 6 is retracted relative to the inner catheter 4. The catheter assembly 8 is guided through the cervix and into the uterus. If the catheter assembly 8 becomes obstructed, the sheath 6 is extended to add rigidity to the distal end of the catheter assembly 8, which allows the medical professional to bypass the obstruction. The zygote can then be implanted in the manner described above.

The above-described prior art techniques for performing IVF are both cumbersome and awkward for several reasons. First, the inner diameter and the total volume of the inner catheter 4 is very small compared to the displacement of the proximal syringe 2. Because of this high differential, it is hard to precisely control the displacement rate (both aspiration and expulsion) of zygotes and growth medium through the inner catheter 4. Even a small displacement of the syringe plunger creates a large volume displacement in the catheter. Therefore, it would be desirable to provide an apparatus that can aspirate and dispense zygotes and growth medium from a IVF Luer catheter under precisely controlled conditions.

Prior art methods of IVF usually require two or more medical professionals to transfer the zygote from the growth medium to the uterine wall. This can be clumsy, especially when working in close proximity to the patient. One medical professional usually controls the syringe while another positions and controls the catheter assembly 8. Therefore, it would be desirable to provide a method and/or apparatus that can aspirate and dispense zygotes and growth medium from a IVF Luer catheter that can be operated by a single medical professional.

Prior art methods also rely heavily on the skill of the medical professional to carefully control the aspiration and expulsion rate through the catheter by precisely controlling depression and extraction of the plunger of the syringe 2. It has been theorized that the rate at which the zygote and growth medium are admitted and expelled from the catheter assembly 8 may affect the success rate, i.e., fertility rate, of the IVF procedure. Therefore, it would be desirable to provide a method and apparatus that transfers zygotes from the growth medium to the uterine wall during IVF under precisely controlled and repeatable conditions and in a less awkward manner.

### Summary of the Invention

The present invention provides devices for performing *in vitro* fertilization ("IVF") to maximize successful fertilization rates by optimizing the conditions under which zygotes are transferred from growth medium to the uterine wall. The invention provides more convenient and scientifically reliable apparatuses for extracting zygotes from growth medium and implanting the zygotes on the uterine wall of a patient.

In accordance with one embodiment of the invention, an apparatus is provided for performing an IVF procedure. The apparatus includes a control unit having an external port and means for creating positive and negative fluid pressure at said port.

The apparatus also includes a flexible extension hose having a first coupling at a proximal end, which can be connected in sealed fluid communication to said port, and a second coupling at the distal end, which can be constructed and arranged to connect to a fitting of a catheter assembly (8). The apparatus also includes an inter-uterine catheter assembly (8) connected to said second coupling. The catheter assembly has an inner catheter having a proximal end, a quick connect/disconnect fitting at the proximal end connectable to said second coupling, and an open distal end; and an outer sheath surrounding and slidable over said inner catheter (4) and having an open distal end. The extension hose includeses a central tubular pneumatic passageway; an inner sheath surrounding and protecting said central tubular passageway; and
an outer sheath surrounding said inner sheath and protecting said central tubular passageway.

The control unit may have a housing and a command display on the housing. An internal pump may be located inside the housing and is connected in pneumatic communication with the external port. The pump is constructed and arranged to create either positive pressure or negative pressure at the port.

The control unit may include a programmable microprocessor that accepts input commands from the display and controls operation of the pump. Optionally, the control unit has remote control means for activating, de-activating, and reversing the flow of the pump. For example, the remote control means may comprise foot switches connected to the microprocessor.

The control unit can be programmed to repeatedly provide the same aspiration and expulsion fluid flow conditions through the catheter assembly. The control unit may also be programmed to provide a defined sequence of aspiration and expulsion fluid flow conditions through the catheter assembly. Optionally, the control unit includes an internal memory that records the fluid flow conditions through the catheter assembly during each step of the IVF procedure.

The hose includes a central, tubular pneumatic passageway, an inner sheath surrounding and protecting the central tubular passageway, and an outer sheath surrounding the inner sheath and protecting the central tubular passageway. Preferably, the central passageway and the inner sheath comprise flexible, polycarbonate tubing, and the outer sheath comprises flexible, silicone tubing.

Preferably, the first and second couplings of the hose have a quick connect/disconnect construction. In one embodiment, the first and second couplings comprise a Luer fitting having a barbed connection stem, an insert having a central, axial aperture, and an adhesive affixing the central passageway in the central aperture of the insert. The ends of the inner sheath, outer sheath and central passageway are connected to the couplings such that pressurized air from the control unit conveys only through the central passageway. The outer surface of the central passageway is adhered inside the central, axial aperture of the insert. The inner sheath is compressed between the outer surface of the central passageway and the inner surface of the central, axial aperture. The ends of the outer sheath surround and connect to the barbed connection stem.

In another embodiment, the apparatus includes a carrier constructed and arranged to support and temporarily lock the catheter assembly in an orientation wherein the outer sheath has been slid axially forward to cover the distal portion of the inner catheter. The carrier has an elongate handle having a longitudinal axis and opposed ends. An elongate tray is fixed to one end of the handle and extends transverse to the longitudinal axis. A distal and proximal socket are fixed at opposed ends of the tray. At least a portion of the interior surface of each socket has a shape that compliments at least a portion of the exterior surface of the quick connect/disconnect fittings of the catheter assembly. At least a portion of the interior surface of the distal socket has a shape that also compliments at least a portion of the outer sheath.

In another embodiment, the apparatus includes an injector constructed and arranged to support and temporarily lock the outer sheath of the IVF catheter assembly in a plurality of positions relative to the catheter, and to extend and retract the outer sheath relative to the catheter. The injector can retract the outer sheath proximally relative to the catheter assembly to a first limit position exposing the distal tip of the inner catheter, and can extend the outer sheath distally relative to the catheter assembly to a second limit position covering the distal tip of the inner catheter. The injector is preferably constructed and arranged to be held and operated using a single hand.

In a preferred embodiment, the injector has a gun-shaped housing including a handle, and a barrel having a distal end and a proximal end relative to the handle. A catheter assembly mount is fixed to and supported by the barrel. A trigger is rotatably connected to the upper portion of the handle A sheath extender and retractor assembly is mounted in the barrel and connected to the trigger.

In one preferred embodiment, the sheath extender and retractor assembly includes an elongate activation rod having a distal portion extending from the distal end of the barrel and a proximal portion slidably mounted in the barrel. An advancement assembly is connected to and activated by the trigger. The advancement assembly engages the proximal portion of the activation rod. A nosepiece is fixed to the distal portion of the activation rod. A retraction knob is fixed to the proximal end of the activation rod.

In one preferred embodiment, the extension hose includes a three-way vent arranged in fluid communication with the central passageway and is fixed proximate the distal coupling.
In this embodiment, the mount comprises a proximal socket formed in the upper surface of the barrel and a distal socket formed in the nosepiece. The shape of the proximal socket compliments the shape of the three-way vent and the distal coupling so that the vent and coupling can temporarily nest in the proximal socket. The shape of the distal socket compliments the shape of the outer sheath so that at least a portion of the outer sheath can temporarily nest in the distal socket. Optionally, the apparatus includes a light fixed to the nosepiece.

Any of the above-described apparatuses or other devices can be used in a method of performing an *in vitro* fertilization ("IVF") procedure comprising the initial step of providing a plurality of human zygotes in a growth medium. Using any of the above-described apparatuses or other devices, zygotes are sequentially aspirated into an IVF catheter and surrounded within the IVF catheter by a column of growth medium. The IVF catheter is inserted into the patient's uterus and the distal tip of the IVF catheter is located proximate the uterine wall. Each zygote is sequentially implanted on the uterine wall by dispensing the zygotes from the IVF catheter.

Preferably, the steps of aspirating and implanting the zygotes are performed using remote control means. The steps of aspirating and implanting each zygote may be performed using pre-programmed, automated control means or hands-free remote control means.

The step of dispensing each zygote from the IVF catheter can be performed using the same fluid flow conditions through the catheter. The step of aspirating each zygote from the growth medium into the catheter can be performed using the same fluid flow conditions through the catheter. These fluid flow conditions are controlled by the control unit.

The method may also include the step of measuring and recording the fluid flow conditions through the catheter during each step of the IVF procedure. Using this data, IVF success rate can be optimized by counting the number of zygotes that successfully develop into embryos, comparing the success rates to the recorded fluid flow conditions, and calculating the optimum fluid flow conditions for an IVF procedure.

Using, for example, any of the above-described apparatuses or other devices, zygotes are sequentially aspirated into an IVF catheter and surrounded within the IVF catheter by a column of growth medium. The IVF catheter is inserted into the patient's uterus and the distal tip of the IVF catheter is located proximate the uterine wall. Each zygote is sequentially implanted on the uterine wall by dispensing the zygotes from the IVF catheter. The number of zygotes that successfully develop into embryos under those fluid flow conditions is then counted. Multiple IVF procedures are performed under different, recorded fluid flow conditions. Finally, a statistical analysis is performed of the data gathered from the multiple IVF procedures to determine the optimum fluid flow conditions for an IVF procedure. This method may include the step of aspirating and dispensing each zygote during the IVF procedure using the same fluid flow conditions.

In another preferred embodiment, an injector is provided for performing an IVF procedure using an inter-uterine catheter assembly. The catheter assembly comprises an inner catheter having a proximal end, a quick connect/disconnect fitting at the proximal end, and an open distal end. The catheter assembly also comprises an outer sheath surrounding and slidable over the inner catheter. The sheath has an open distal end and a fitting at a proximal end. The injector is constructed and arranged to support and temporarily lock the outer sheath in a plurality of positions relative to the catheter, and to extend and retract the outer sheath relative to the catheter.

In a preferred embodiment, the injector can retract the outer sheath proximally relative to the catheter assembly to a first limit position exposing the distal tip of the inner catheter, and can extend the outer sheath distally relative to the catheter assembly to a second limit position covering the distal tip of the inner catheter. The injector is preferably constructed and arranged to be held and operated using a single hand. The injector has the same construction as described above with respect to the IVF apparatus.

In another preferred embodiment of the invention, an apparatus is provided for performing an in vitro fertilization ("IVF") procedure using a standard Luer catheter assembly. The apparatus includes a control unit 12 having a housing with an external port, and a command display on the housing. An internal pump is connected in pneumatic communication with the external port. The pump is constructed and arranged to create either positive pressure or negative pressure at the port. A control unit includes a programmable microprocessor that accepts input commands from the display and controls operation of the pump.

A flexible extension hose is connected to the control unit. The flexible hose has a first coupling at a proximal end, which is coupled to the port on the control unit. The hose has a second coupling at the distal end, which is constructed and arranged to connect to the Luer fitting of a Luer catheter assembly.

The apparatus includes remote control means for activating, de-activating, and reversing the flow of the pump. The remote control means may comprise foot switches connected to the microprocessor. The control unit can be programmed to repeatedly provide the same aspiration and expulsion flow conditions within the catheter assembly. The control unit can also be programmed to provide a defined sequence of aspiration and expulsion fluid flow conditions through the catheter assembly. Preferably, the apparatus includes an internal memory that records the fluid flow conditions through the catheter assembly during each step of the IVF procedure.

### Brief Description of the Drawings

Fig. 1 is a perspective view of a prior art zygote transfer catheter connected to a syringe for performing IVF;
Fig. 2 is a perspective view of an IVF control apparatus in accordance with an embodiment of the invention;
Fig. 3 is an enlarged, fragmentary perspective of the extension tube shown in Fig. 2 in accordance with an embodiment of the invention;
Fig. 4 is an enlarged, fragmentary perspective of one end of the extension tube shown in Fig. 3;
Fig. 5a is an exploded perspective view of one end of the extension tube shown in Figs. 3 and 4;
Fig. 5b is an exploded, side elevational view of one end of the extension tube shown in Figs. 3 and 4;
Fig. 6a is a perspective view of the insert shown in Fig. 5;
Figs. 6b and 6d are front and back elevational views of the insert show in Fig. 6a;
Fig. 6c is a side elevational view of the insert shown in Fig. 6a;
Fig. 7a is a fragmentary, side elevational view of the inner tubing and inner sheath of the extension hose connected to the insert;
Fig. 7b is an enlarged, cross-sectional view taken along lines A-A of Fig. 7a;
Fig. 7c is an enlarged, fragmentary view of section B of Fig. 7b;
Fig. 8a is a fragmentary side elevational view of one end of the extension hose and a coupling as shown in Fig. 3;
Fig. 8b is an enlarged, cross-sectional view taken along lines A-A of Fig. 8a;
Fig. 8c is an enlarged, fragmentary view of section B of Fig. 8b;
Fig. 9a is a perspective view of the control unit of Fig. 3;
Fig. 9b is a schematic diagram of main components within the control unit of Fig. 3;
Fig. 10 is a perspective view of a transfer carrier for holding the zygote transfer catheter assembly in accordance with an embodiment of the invention;
Fig. 11 is an enlarged, fragmentary view of a zygote transfer catheter assembly mounted in the transfer carrier of Fig. 10;
Fig. 12 is a perspective view of an IVF control apparatus in accordance with another embodiment of the invention;
Fig. 13 is an enlarged perspective view of the injector shown in Fig. 12;
Fig. 14 is a perspective view of the injector with the front housing component removed;
Fig. 15 is a top plan view of the injector shown in Fig. 12;
Fig. 16 is an enlarged, fragmentary perspective view of the distal end of the injector;
Fig. 17 is a cross-sectional view of a catheter assembly mounted on the injector shown in Fig. 12 taken along the median plane of the injector;
Fig. 18 is an enlarged, fragmentary perspective view of the rod resistance assembly of the injector;
Fig. 19 is schematic illustration (not to scale) of the activation tab of the sheath extender and retractor assembly; and,
Fig. 20 is an enlarged, fragmentary perspective view of the activation tab portion of the injector.

### Description of Preferred Embodiments of the Invention

For the purpose of illustrating the invention, several embodiments of the invention are shown in the accompanying drawings. However, it should be understood by those of ordinary skill in the art that the invention is not limited to the precise arrangements and instrumentalities shown therein and described below. Throughout the specification, like reference numerals are used to designate like elements. Throughout the specification, as used in connection with various elements and portions of elements, the terms "distal" and "proximal" refer to their spatial relationship relative to the control unit.

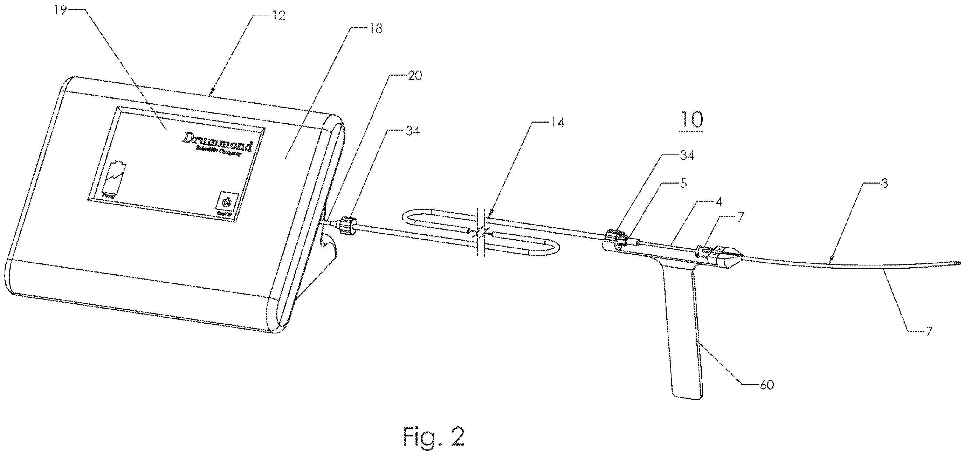

An IVF controller apparatus in accordance with a preferred embodiment of the invention is schematically illustrated in Fig. 2. The IVF controller, designated generally by reference numeral 10, generally comprises a control unit 12 having an external port 20 and means for creating positive and negative fluid pressure at said port 20, and an elongate, flexible extension hose 14 having a first coupling 34 at a proximal end 14a, which can be connected in sealed fluid communication to said port 20, and a second coupling 34 at the distal end 14b, which can be constructed and arranged to connect to a Luer fitting 5 of a Luer catheter assembly 8 as illustrated in Fig. 2. In one embodiment of the invention, the IVF controller 10 connects to a standard, inter-uterine zygote catheter assembly 8 such as shown in Fig. 1. The catheter assembly 8 comprises an inner catheter 4 having a Luer fitting 5 at the proximal end 4a and an open distal end 4b. The catheter assembly 8 also has an outer sheath 6 having a Luer fitting 7 at the proximal end 6a and an open distal end 6b. In another embodiment, the IVF controller includes a novel inter-uterine zygote catheter having a similar construction but specially designed to releaseably connect to the extension hose 14.

In the embodiment shown in Fig. 2, a prior art catheter assembly 8 maybe connected in fluid communication with the control unit 12 through the flexible pressure hose 14. The control unit 12 selectively creates either negative or positive pressure at the distal opening 4b of the inner catheter 6 for extracting zygotes from culture medium, and implanting the zygotes on the uterine wall of the patient.

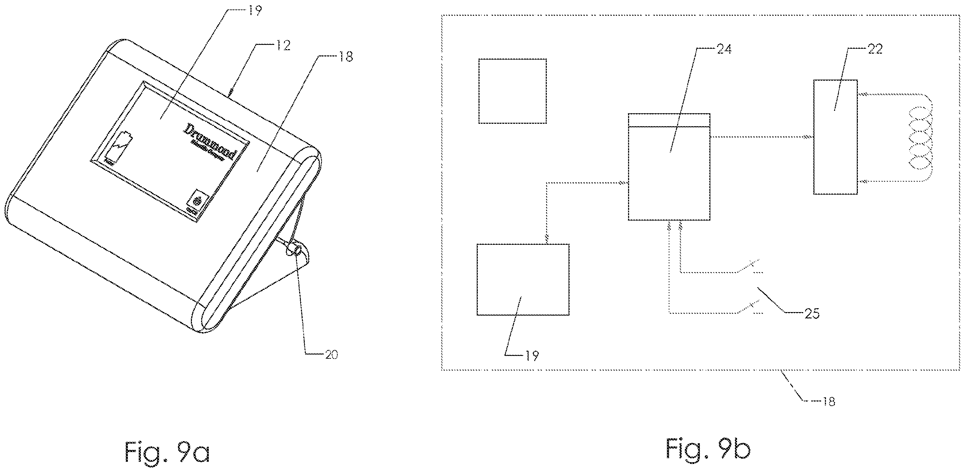

A control unit 12 in accordance with an embodiment of the invention is shown in Figs. 2 and 9. In this embodiment, the control unit generally comprises a housing 18, a touch-screen display 19 on the housing 18, an external pneumatic connection port 20, and an internal pump 22 that is connected in pneumatic communication with the port 20. The pump 22 can create either positive pressure or negative pressure at the port 20 using known techniques. The control unit preferably includes a programmable microprocessor 24, which can accept user commands entered on the display 19. For example, the user may command the motor to turn on/off, run at a defined speed, run in a particular mode (positive or negative pressure), run in a defined cycle, etc. The commands and past activity of the controller may be stored in an internal memory 26. Optionally, the control unit 12 has foot switches 25 connected to the microprocessor, which control activation and de-activation of the pump.

The external port 20 is designed to releasably connect to the proximal end of the extension hose 14. In this embodiment, the port 20 has external threading that engages the internal threading of the coupling 34 fixed to the proximal end 14a of the extension hose 14 (described below).

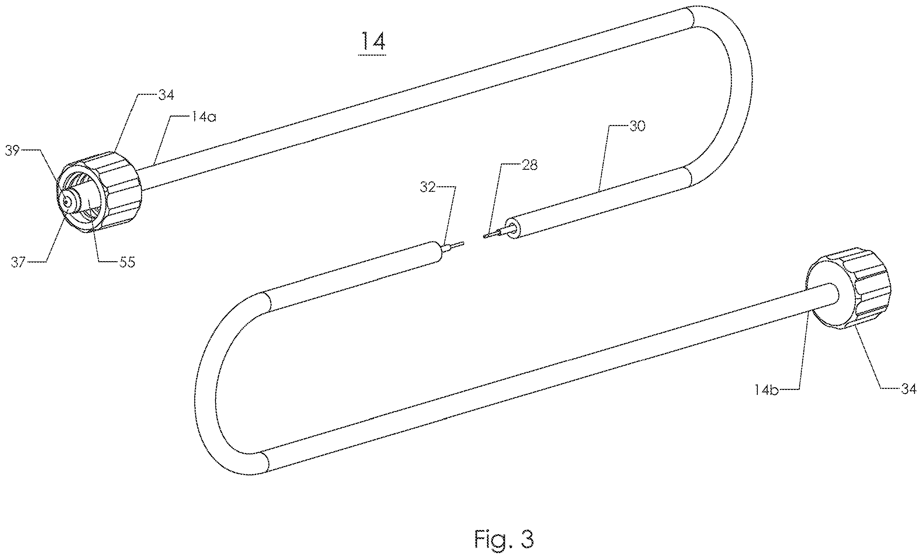

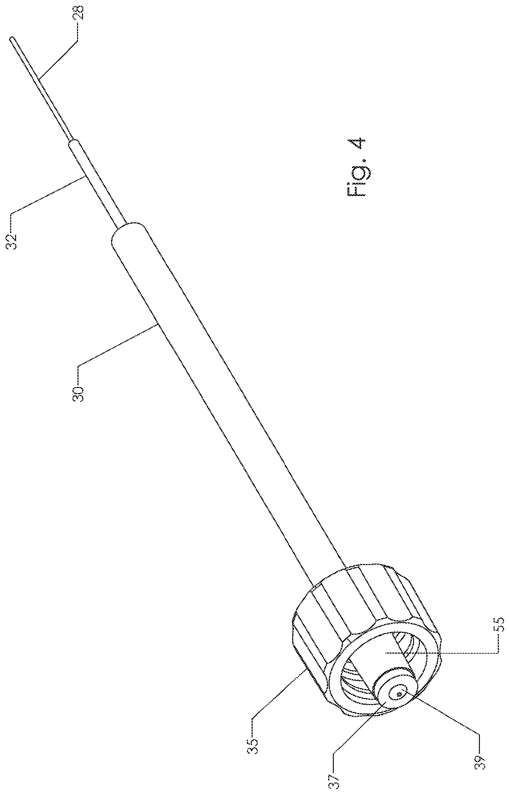

An extension hose 14 in accordance with an embodiment of the invention is shown in greater detail in Figs. 3-8. In a preferred embodiment, the hose 14 includes a central, tubular pneumatic-communication passageway 28. In a preferred embodiment, the passageway 28 comprises flexible, polycarbonate tubing. In the embodiment shown in Figs. 3-8, the central tubing has an inner diameter (ID) of about 0,254mm (0,01 inch) and an outer diameter (OD) of about 0,381mm (0,015 inch). It is preferred to minimize the ID and tube length so that the amount of unoccupied or "dead space" within the tube is also minimized.

The hose 14 also includes an outer sheath 30, which provides a protective barrier for the central tubular passageway 28 and a secure connection between the control unit 12 and the catheter assembly 8. In a preferred embodiment, the outer sheath 30 comprises flexible, silicone tubing having an inner diameter larger than the outer diameter of the central tubing 28 and an outer diameter that makes handling easy for the user and tough enough to withstand normal handling in the laboratory or procedure room. In the embodiment shown in Figs. 3-8, the outer sheath 30 has an ID of about 1,5875 mm (0,0625 inch) and an outer diameter of about 3,175mm (0,125 inch). In this embodiment, the outer sheath 30 comprises common (stock) silicone tubing that is designed to reliably connect to a Luer fitting, described below.

In a preferred embodiment, the hose 14 includes a second or inner sheath 32. In this embodiment, the inner sheath 32 comprises polycarbonate tubing having an inner diameter slightly larger than the outer diameter of the central tubing 28 and an outer diameter slightly smaller than the inner diameter of the outer sheath 30. In a preferred embodiment, the inner sheath 32 has an ID of about 0,5588 mm (0,022 inch) in. and an OD of about 0,889 mm (0,035 inch). The inner sheath 32 provides structural integrity and ease of integration with a standard Luer fitting.

In a preferred embodiment, the hose 14 has a quick connect/disconnect coupling 34 at each end. In the embodiment shown in Figs. 3-8, the coupling has the same construction at both ends but slightly different dimensions; however, depending on the configuration of the port 20 on the control unit 12 or the configuration of the catheter assembly 8, the couplings 34 could have different constructions.

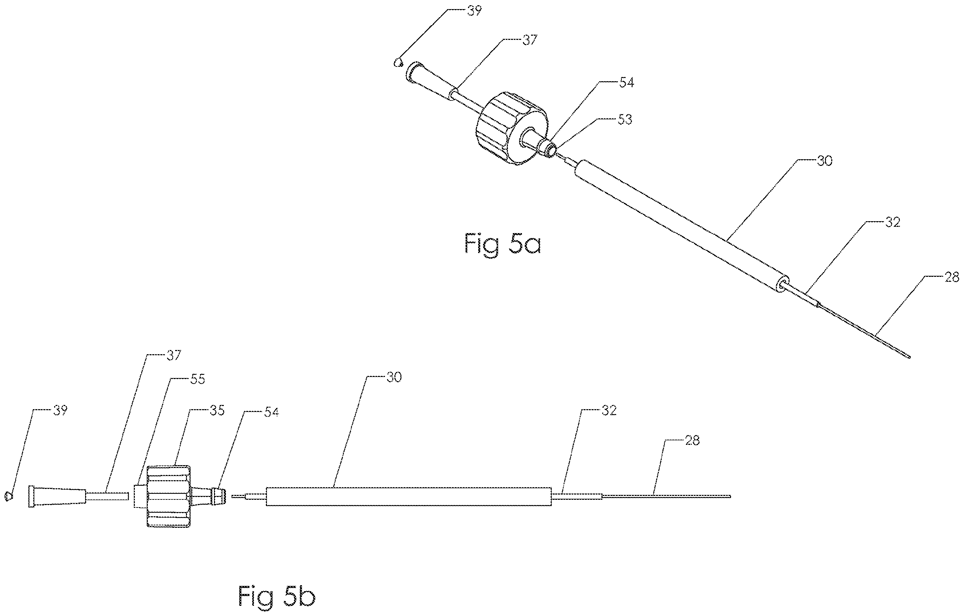

Referring to Figs. 5a and 5b, in a preferred embodiment of the invention, the coupling 34 comprises a Luer fitting 35, an insert 37 for the Luer fitting, and an adhesive 39 such as UV light-curable adhesive. The ends of each of the sheaths 30, 32 and the central passageway 28 are connected to the couplings such that pressurized air conveys only through the central passageway 28.

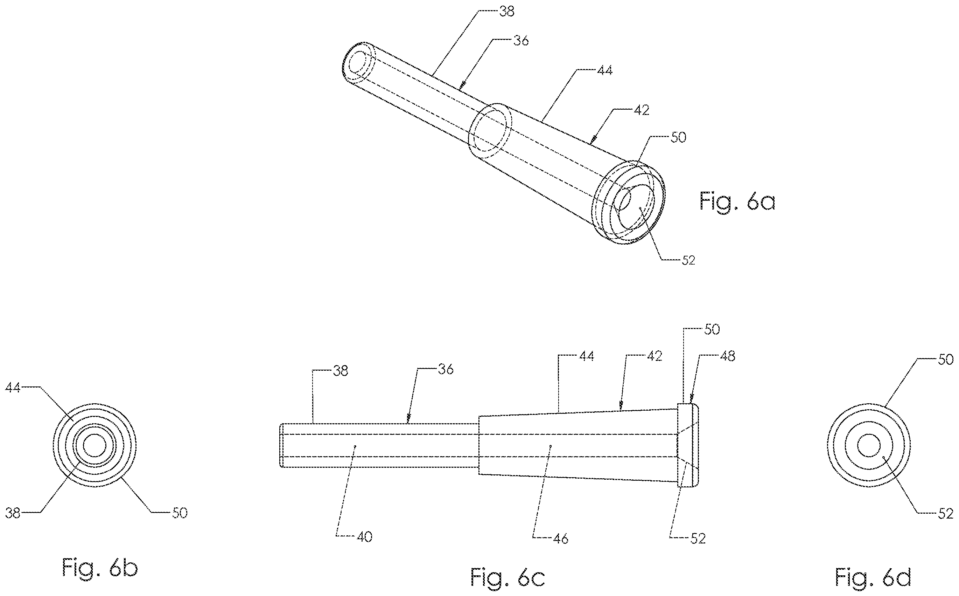

Referring to Figs. 6a-c, the insert 37 has an elongate, irregular shape comprised of three, integrally-formed sections, each having regular geometries. The first section 36 is cylindrical with a constant-diameter outer surface 38 and a constant-diameter inner bore 40. The second (intermediate) section 42 has a frusto-conical outer surface 44 and a constant-diameter inner bore 46. The third section 48 resembles an annular collar with a constant-diameter outer surface 50 larger than the largest outer diameter of the second section 42. The third section 48 also has a tapered bore 52 connected to the bore 46 of the second section 42. All three bores, 40, 46, and 52 are coaxial. The insert greatly reduces the fluid flow opening through the couplings, which is important for precisely controlling the fluid flow condition through the catheter of the catheter assembly.

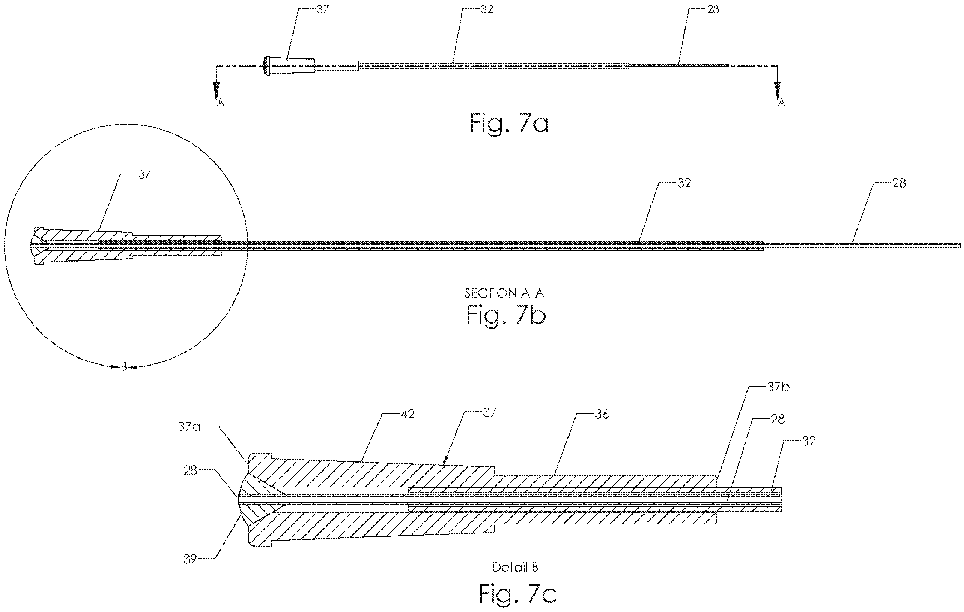

The connection between the extension hose 14 and the coupling 34 is illustrated in greater detail in Figs. 7a - 8c. Referring to Figs. 7a-c, the central tubing 28 extends entirely through the central bore of the insert 37 and is sealed to the distal end 37a by an adhesive 39. The adhesive does not occlude the opening at the end of the passageway 28. The inner sheath 32 extends midway through the central bore to an intermediate position within the second portion 42 of the insert 37. The inner sheath 32 may be affixed in this position by adhesive or a force fit wherein the inner sheath is compressed between the outer surface of the central tubing and the inner surface of the central bore of the insert 37.

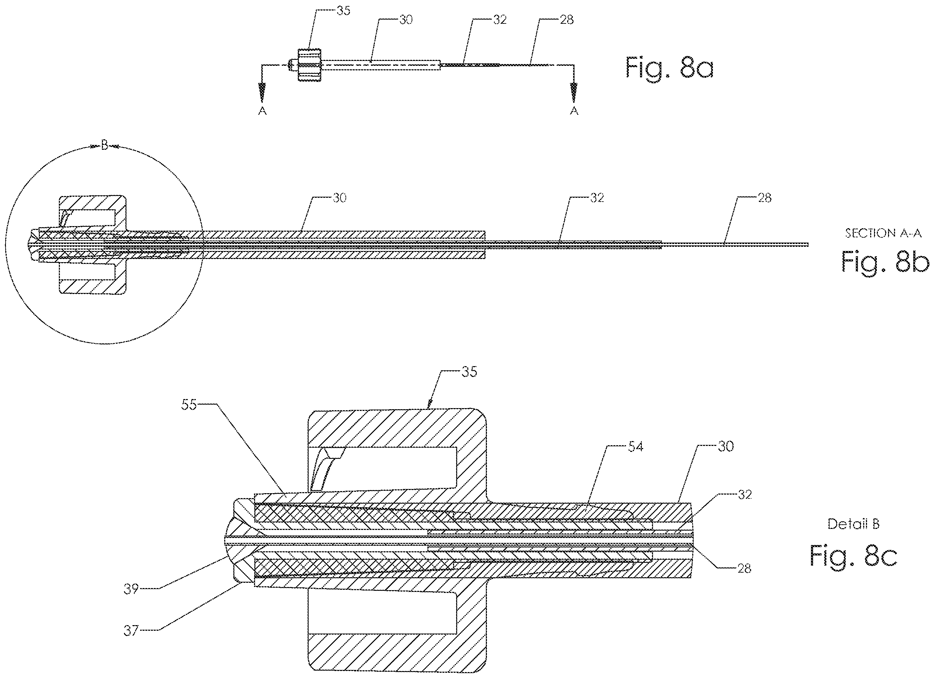

Referring to Figs. 5a, 5b, and 8a-c, the Luer fitting 35 has a central bore 53 extending from the hose barb portion 54 to a connection port 55 on the opposite end. The outer sheath 30 is connected and sealed to the hose barb portion 54 of the Luer fitting 35. The insert 37 engages and seals the connection port 55 of the Luer fitting. As a result of this construction, the central passageway 28 provides the only pneumatic communication channel through the Luer fitting 35, thereby effectively reducing the volume of the central passageway through the Luer fitting compared to the volume of the passageway provided by a flexible silicone tube attached to the hose barb 54 of the Luer fitting. The inner and outer sheaths 30, 32 encase the inner passageway 28, provide structural integrity and ease of integration with a standard Luer fitting 35.

In a preferred embodiment, the distal end of the extension hose 14 is connected (threaded) to an inter-uterine, zygote catheter assembly 8 via the distal coupling 34. In the embodiment shown in Figs. 2-10, the distal coupling 34 is configured to engage the proximal Luer fitting 5 of the catheter assembly 8. The IVF controller 10 can then be used to transfer zygotes during IFV in a more controlled and convenient manner. Instead of manipulating a syringe 2 to aspirate and expel zygotes and growth medium from the catheter assembly 8, the medical professional need only program the control unit 12 with the desired fluid flow conditions including flow rate, pressure, pause time (if desired), turn off time, etc. Alternatively, or additionally, the medical professional can control aspiration and expulsion through the catheter assembly 8 using remote control means such as the foot switches 25. A single medical professional can then easily pick up, transfer and implant numerous zygotes since fluid flow is controlled by the control unit 12 or the remote control means. The professional can set the control unit 12 for a very slow or gentle volumetric fluid flow rate, a rapid flow rate, or something in between. A single professional can then use both hands to manipulate the catheter assembly 8 during the IVF procedure.

In another aspect of the invention, medical professionals can use the IVF controller to optimize fertilization rates. In this aspect of the invention, medical professionals can perform a statistical analysis of success rates of implantation when compared to the controlled conditions under which the zygotes were harvested and implanted. Since the control unit can be programmed to provide the same transfer and delivery conditions within the catheter, the medical professional can record and statistically analyze such conditions to determine whether such delivery conditions have any effect on the success of an IVF procedure. For example, it is theorized that a rapid delivery rate through the catheter assembly 8 can have adverse affects on the zygote. The claimed apparatus allows a professional to repeat the IVF procedure under controlled, constant conditions, such as volumetric flow rate, to study this affect. The prior art does not allow such a study since the user input of the medical professional is variable and cannot be quantitatively measured and recorded. For example, the flow rate used by each professional during harvesting and implantation cannot be measured or reliably reproduced.

In another embodiment of the invention, the IVF controller may include a zygote catheter that is specially designed to connect to the distal end of the extension hose 14. In this embodiment, the design of the controller 10 may be similar to the controller described above except that the distal coupling 34 of the extension hose 14 and the proximal coupling of the catheter must be compatible.

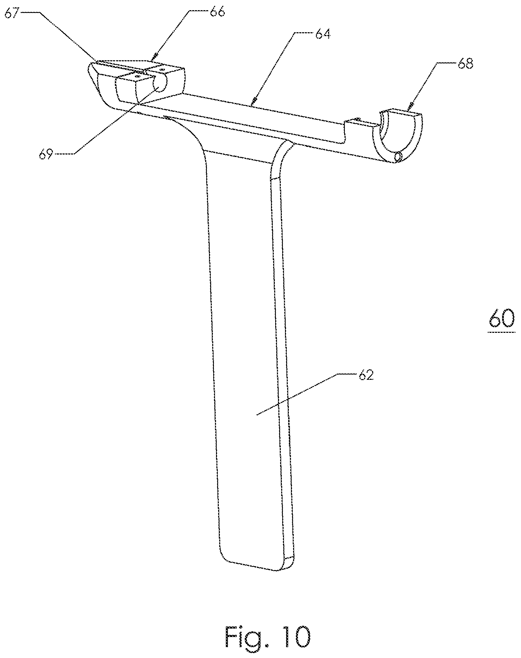

In another embodiment of the invention, the IVF controller includes a hand-held carrier 60 as shown in Figs. 2 and 10. Referring to Fig. 10, the carrier has a handle 62 and a transversely-extending, elongate tray 64 fixed to the upper end of the handle. The tray 64 includes a first or distal socket 66 and a second or proximal socket 68 fixed at opposed ends. As best seen in Fig. 2, the holder is configured to support the catheter assembly 8 in an orientation wherein the outer sheath 6 has been slid axially forward to cover the distal portion of the inner catheter 4. The catheter assembly 8 is typically arranged in this orientation once the zygotes have been extracted from the growth medium and are being transferred to the situs of implantation within the patient's uterus. The carrier 60 locks the catheter assembly in this orientation while the medical professional single-handedly transports the catheter assembly 8 to the implant situs.

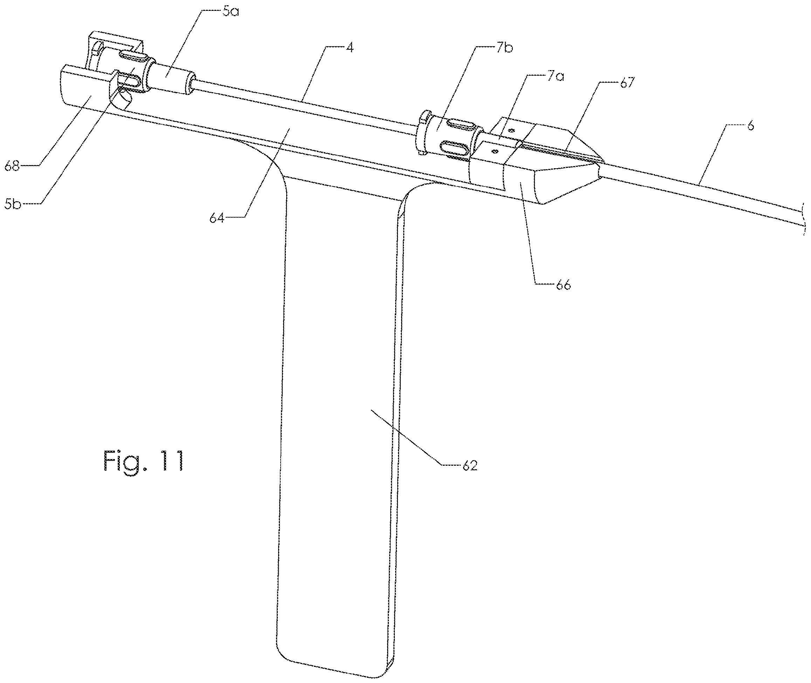

Referring to Figs. 10 and 11, an irregularly-shaped slot extends lengthwise (relative to the tray) through the distal socket 66. The forward portion 67 of the slot has the shape of a half pipe and a width slightly greater than the outer diameter of the sheath 6. The shape of the forward portion 67 enables the sheath 6 to be inserted therein through the lengthwise-extending opening since the width of the opening is larger than the outer diameter of the sheath 6. The rear portion 69 of the slot has the same width on the upper surface as the forward portion 67, but has an inner radius greater than the width on the upper surface. This increased inner radius enables the distal portion 7a of the Luer fitting (fixed to the sheath) to be inserted therein through the end of the socket 67; however, since the width of the slot on the upper surface of the socket 67 is less than the outer diameter of the distal portion 7a of the Luer fitting, the fitting 7 is held in place.

The proximal socket 68 has a generally half-pipe configuration with an inner diameter approximately equal to the outer diameter of the proximal portion 5b of the Luer fitting that is fixed to the catheter 4 of the catheter assembly 8. The inner profile of the proximal socket 68 has a profile that compliments the outer profile of the Luer fitting so that the Luer fitting 35 is held snugly in the proximal socket 68. Thus, the two sockets 66, 68 lock the Luer fittings 5 and 7 at a fixed distance from one another so that the zygotes are not disturbed within the catheter assembly 8.

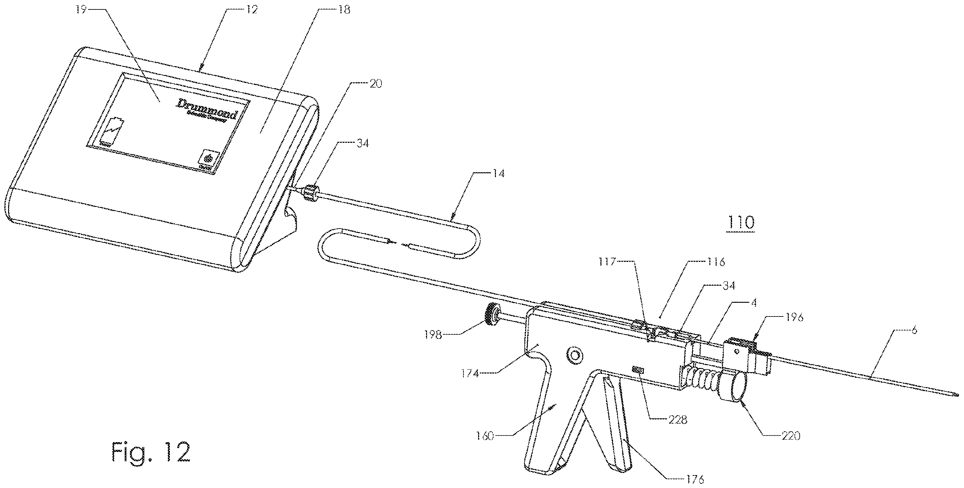

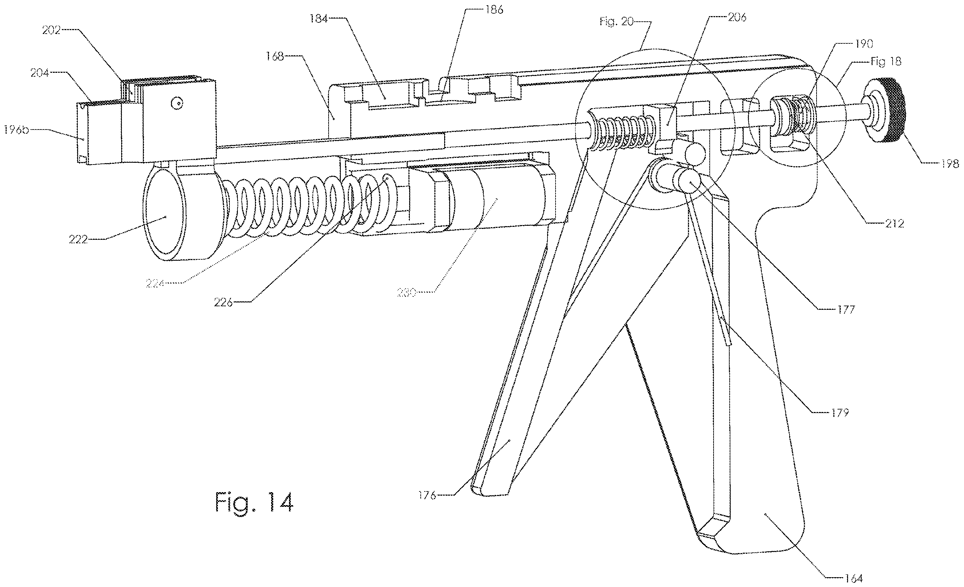

An IVF controller in accordance with another preferred embodiment of the invention is illustrated in Figs. 12-20 and is designated generally by reference number 110. In this embodiment, the IVF controller 110 includes a control unit 12, which has the same construction and functionality as described above. In an alternative embodiment, the controller 110 also includes a second control unit that is designed exclusively to dispense fluid from the catheter assembly 8. In this embodiment, the first control unit 12 is located in the laboratory where the catheter assembly 8 is loaded with zygotes. The second control unit is located in the operating/procedure room. The second control unit has limited functionality (no aspiration capability) since aspiration into the catheter assembly 8 does not occur during the uterine implant phase of the IVF procedure.

The IVF controller 110 also includes an extension hose 114, which has substantially the same construction as the extension hose 14 described above; however, in this embodiment, the extension hose 114 includes a three-way vent 116 connected intermediate the distal end of the hose 114 and the distal Luer connector 34. In this embodiment, the extension hose 114 is designed to connect to an inter-uterine, zygote catheter assembly 8 such as a standard inter-uterine zygote catheter assembly 8 shown in Fig. 1.

The IVF controller 110 of this embodiment includes a hand-held injector 160. In this embodiment, the injector 160 replaces the hand-held carrier 60 shown in Figs. 1-11 and provides increased functionality compared to the carrier 60. As best seen in Figs. 12, 16, and 17, the injector 160 is configured to support the catheter assembly 8 in a plurality of configurations ranging from a first limit position, wherein the outer sheath 6 has been slid axially rearwardly to expose the distal tip of the inner catheter 4, to a second limit position wherein the outer sheath 6 has been slid axially forwardly to cover the distal portion of the catheter 4. Similar to the carrier 60, the injector 160 locks the catheter assembly 8 in the preferred configuration while the medical professional single-handedly transports the catheter assembly 8 to the implant situs. Furthermore, as described below, the injector 160 enables the medical professional to incrementally extend the outer sheath 6 relative to the inner catheter 4 once it has been inserted in the patient's uterus.

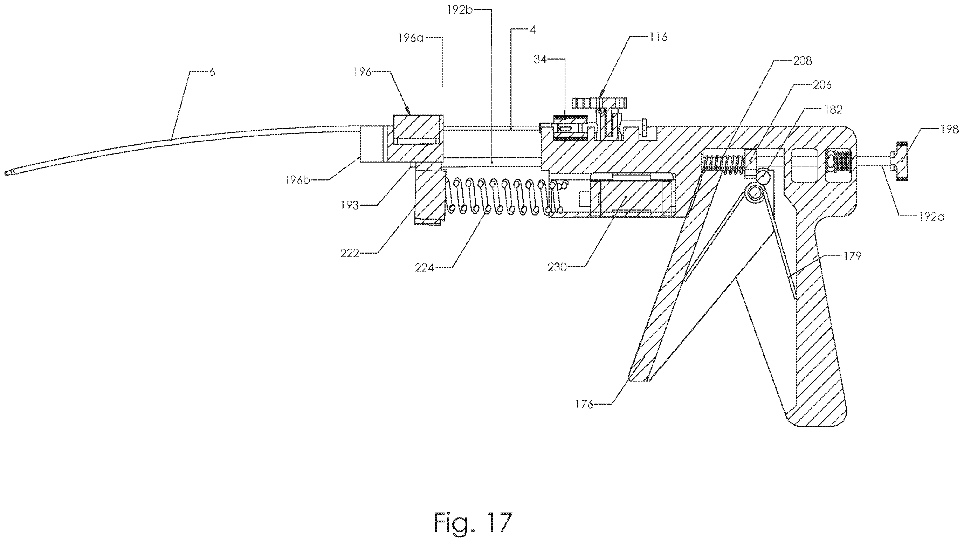

The injector 160 has a gun-shaped housing 162 including a handle 164, and a barrel 166 having a distal end 168 and a proximal end 170 relative to the handle 164. In a preferred embodiment, the housing 162 has an internal cavity formed from two, generally-symmetrical housing halves that connect along an interface coplanar with the median plane of the injector 160, which divides the injector into a left side 172 and a right side 174 (relative to a line of sight looking down the barrel from the proximal end 170 to the distal end 168). A trigger 176 is rotatably connected with a pivot pin 177 to the upper portion of the handle 164 and rotates in the median plane of the housing 162. A coil spring 179 normally biases the trigger 176 to an extended position (relative to the handle) as best seen in Fig. 17. The lower portion of the trigger 176 preferably has a shape that compliments the human hand. As the trigger 176 is depressed and released relative to the handle 162, an activation pin 182 in the upper portion of the handle 162 is driven forward then backward, respectively, within the barrel 166. As described below, the activation pin 182 engages an activation tab 206 of a sheath extender and retractor assembly 190. The housing 162 and trigger 176 are preferably made from a lightweight polymer using known molding techniques.

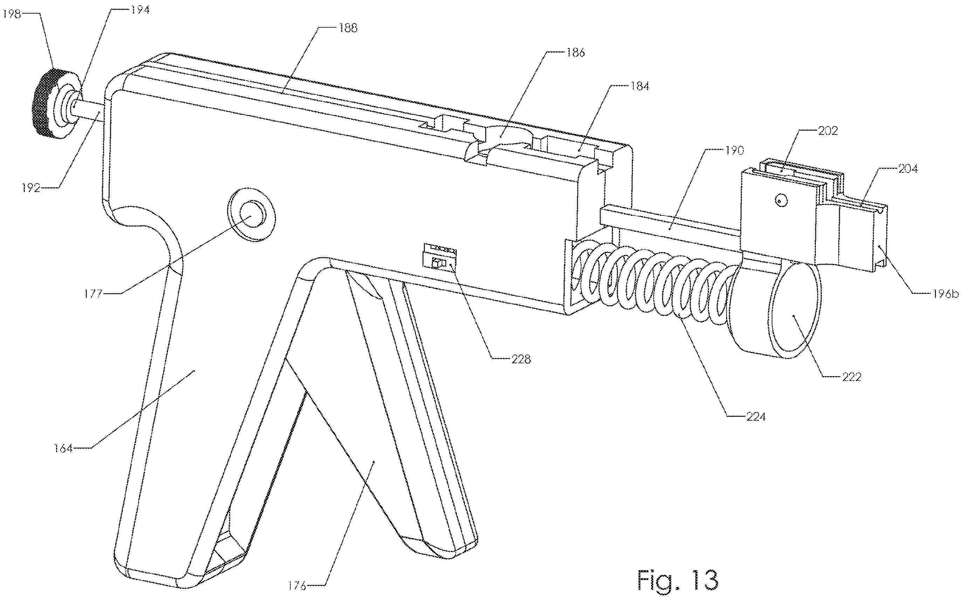

Referring to Figs. 13-15, two recesses and a channel are formed in the upper, exterior surface of the barrel 166. The first recess 184 is located proximate the distal end 168 of the barrel 166 and has a shape that compliments the shape of a Luer fitting. The second recess 186 is located proximate the first recess 184 and has a shape that compliments the shape of the three-way vent 116. The channel 188 extends from the second recess 186 to the proximal end 170 of the barrel 166 and has a shape that compliments the extension hose 114. The recesses 184, 186 and channel 188 form an irregularly-shaped socket in which the catheter assembly 8 and distal end of the extension hose 14 can be temporarily seated during the IVF procedure as seen in Figs. 12, 16 and 17. The dimensions of the first and second recess 184, 186 and channel 188 are slightly smaller than the outer dimensions of the Luer fitting 5, three-way vent 116, and extension tube 114, respectively, so that the inter-uterine catheter assembly 8 and distal end of the extension hose 114 fit snugly in the socket.

A sheath extender and retractor assembly, designated generally by reference numeral 190, is mounted in the barrel 166 of the injector 160. The sheath extender and retractor assembly 190 enables the technician to extend and retract the outer sheath 6 of the catheter assembly 8 by depressing the trigger 176 and retracting the retraction knob 198, respectively.

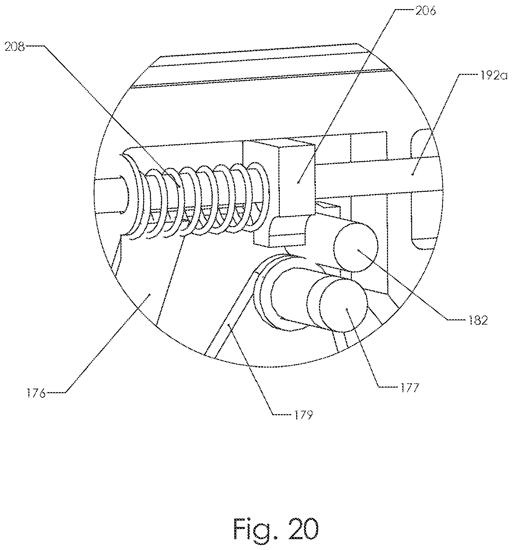

The extender and retractor assembly 190 has an elongate rod 192 that extends through the entire length of the barrel 166. A nosepiece 196 is fixed to the distal end 193 of the rod 192. A retraction knob 198 is fixed to the proximal end 194 of the rod 192. In a preferred embodiment, the proximal portion 192a of the rod 192 has a circular cross-section while the distal portion 192b of the rod 192 has a square cross-section as best seen in Fig. 20. In this embodiment, the diameter of the proximal portion 192a is approximately the same as the width of the distal portion 192b so that the entire length of the rod 192 can slide within a square channel 200 extending (partially) through the barrel 166. The square distal portion 192a of the rod 192 and the square channel 200 prevent the rod 192 from rotating axially during transverse movement.

The nosepiece 196 has an irregular shape and is formed from two generally-symmetrical component halves that connect along an interface coplanar with the median plane of the injector 160, which divides the nosepiece into a left side and a right side (relative to a line of sight looking down the barrel 166 from the proximal end 170 to the distal end 168). The nosepiece 196 has a recess 202 formed in the proximal end 196a, which has a shape that compliments the shape of a Luer fitting. A channel 204 in the nosepiece 196 extends from the recess 202 to the distal end 196b of the nosepiece 196 and has a shape that compliments the sheath 6 of the catheter assembly 8. The channel 204 and recess 202 form an irregularly-shaped socket in which the outer sheath 6 and the Luer fitting 7, respectively, can be temporarily seated during the IVF procedure as seen in Figs. 12, 16 and 17. The dimensions of the channel 204 and recess 202 are slightly less than the outer dimensions of the outer sheath 6 and the Luer fitting 7, respectively, so that they fit snugly in the socket. The nosepiece 196 is preferably made from a lightweight polymer using known molding techniques.

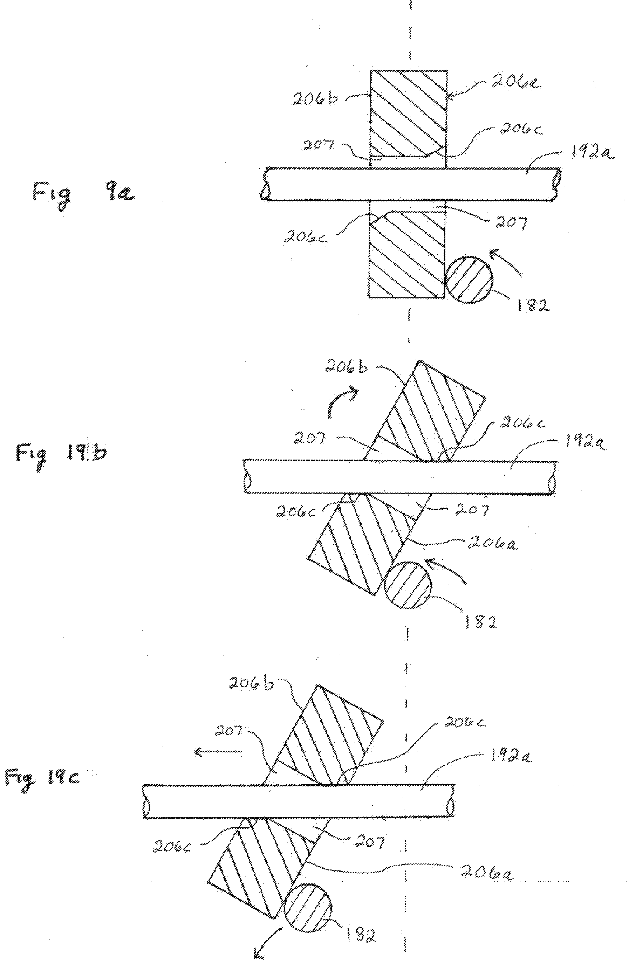

The nosepiece 196 is extended by repeatedly depressing and releasing the trigger 176. As best seen in Fig. 17, a spring-biased activation tab 206 is slidably mounted on the proximal portion 192a of the activation rod 192. Referring to Figs. 19a-c, the rod 192 extends through a central bore 207 extending from the proximal face 206a to the distal face 206b. The bore 207 has two chamfered sections 206c, one each at the upper proximal face and the lower distal face. Referring to Fig. 17, the tab 206 is urged rearwardly by a compression spring 208 into contact with the drive pin 182. Similar to the activation tab 206, the compression spring 208 is also slidably mounted on the activation rod 192.

A portion of the extender and retractor assembly 190 is shown schematically in Figs. 19a-c (not to scale) in three different positions as it gradually advances the activation rod 192 forward. Fig. 19a shows the tab 206 in a first limit position wherein the trigger 176 is fully extended. In this first limit position, the drive pin 182 contacts the lower proximal face 206a of the tab 206. As the trigger 176 is initially depressed (rotated counter-clockwise), the drive pin 182 rotates counter-clockwise, and drives (rotates) the activation tab 206 clockwise to a second position wherein the chamfered corners 206c of the tab 206 contact the activation rod 192 as seen in Fig. 19b. As the trigger 176 is further depressed, the drive pin 182 can no longer rotate the tab 206. Instead, the drive pin 182 pushes the tab 206 forward, which in turn, pushes the rod 192 forward due to the contact friction between the chamfered surfaces 206c of the tab 206 and the rod 192 as seen in Fig. 19c. Once the trigger 176 is released, forward linear movement of the tab 206 and rod 192 terminates. The compression spring 214 then rotates the tab counter-clockwise back to the position shown in Fig. 19a out of frictional engagement with the rod 192. The pitch of the rod 192 during one depression/release cycle of the trigger 176 is very small. Therefore, the rod 192 can be advanced in small, precise increments by repeatedly depressing and releasing the trigger 176.

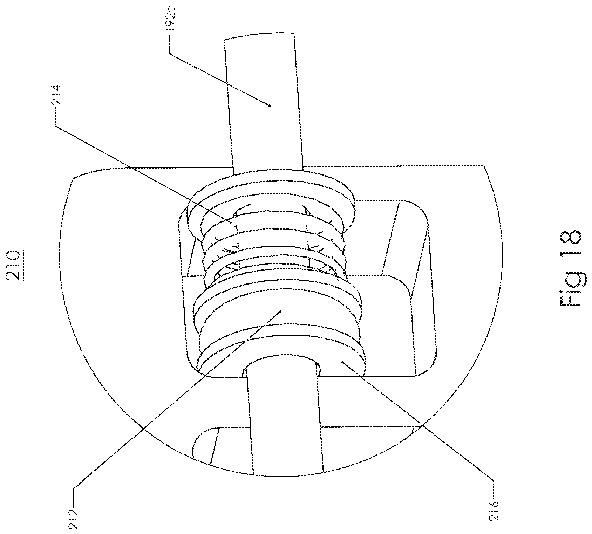

To prevent unintended back lash (reverb) during extension of the rod 192, the injector 160 includes a rod resistance assembly, designated generally by reference numeral 210. The resistance assembly 210 surrounds the proximal portion 192a of the rod 192 and is located within the proximal end portion of the barrel 166. Referring to Fig. 18, the resistance assembly 210 includes an O-ring 212, compression spring 214, and plurality of washers 216. The O-ring preferably comprises an elastomeric material having an inner diameter slightly smaller than the outer diameter of the rod 192 so that the rod 192 is snugly compressed by the O-ring 212 but, at the same time, allows the rod 192 to slide through the O-ring 212 in response to a moderate pushing or pulling force.

A retraction knob 218 is fixed to the proximal end 192a of the activation rod 192. The knob 218 enables the medical professional to retract the activation rod 192 by pulling the knob rearwardly. In the preferred embodiment, the compression spring 214 is located on the proximal side of the O-ring 212, each of which is flanked by a washer 216 as best seen in Fig. 18. Because of this arrangement, the compression spring 208 on the activation tab 206 provides no reverb or kick back when the rod 192 is advanced forward. However, in combination with the compression spring 208 of the sheath extender and retractor assembly 190, the compression spring 214 of the resistance assembly 210 holds the rod 192 stationary relative to the barrel 166 unless a forward activation force is applied by the trigger 176 or a rearward retraction force is applied to the retraction knob 218.

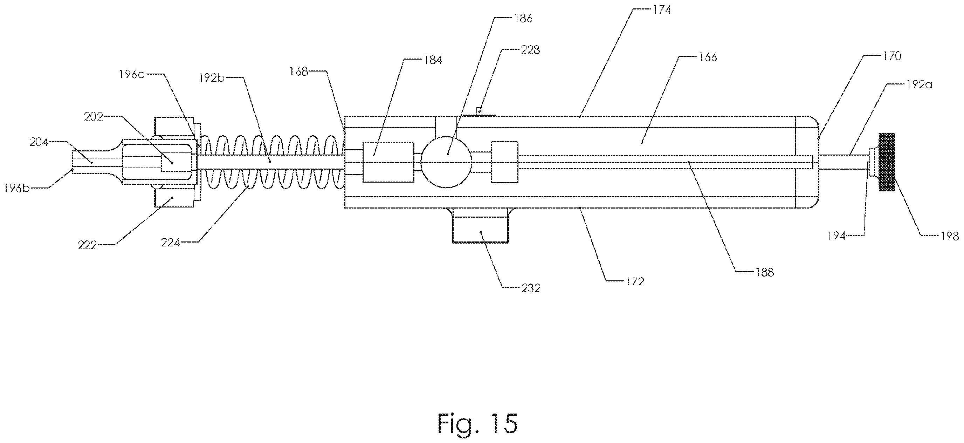

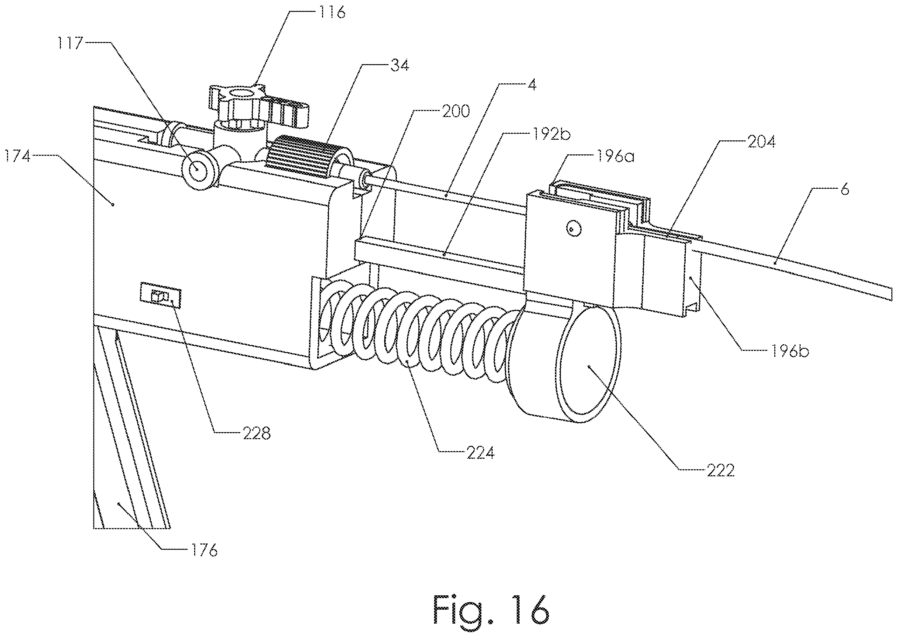

The injector preferably but optionally includes a light assembly, designated generally by reference numeral 220, mounted on the front of the barrel 166. In the embodiment shown in Figs. 12-20, the light assembly 220 comprises an LED bank 222 fixed to the distal end 193 of the rod 192, circuit board 226 and battery 230 located inside the barrel, and a flexible cord 224 connecting the LED bank 222 to the battery 230 and circuit board 226. An on-off switch 228 extends from the right side of the housing 162 as best seen in Figs. 12 and 13. A battery door 232 is located on the left side of the housing 162 as best seen in Figs. 15 and 16. Because the distal portion 192b of the rod 192 slides within a square channel 200, the rod 192, and components connected thereto, cannot rotate about its longitudinal axis. As a result, the nosepiece 196 and light assembly 220 always remain fixed in the proper angular orientation relative to the barrel 166 and focused on the distal tip 4b of the catheter assembly 8 .

In accordance with a preferred method for performing IVF, an inter-uterine, zygote catheter assembly 8 is connected to the distal coupling 34 of the extension hose 14. In the embodiment shown in Figs. 12-20, the distal coupling 34 is connected to the distal port on the three-way valve 116. The proximal coupling is connected to the fluid connection port 20 on the control unit 18. The three-way valve is initially configured to an "open" position wherein the fluid-flow passage between the catheter assembly 8 and the extension hose 14 is open and the passage between the extension hose 14 and the side vent port 117 is closed. The IVF controller assembly 110 is then used in the laboratory to transfer zygotes by sequentially aspirating growth medium and zygotes into the distal end 4b of the inner catheter 4 in a controlled and convenient manner. The medical professional can either program the control unit 12 to aspirate in a controlled sequence, or can personally control aspiration by selectively activating the pump via the foot switches 25.

After the inner catheter 4 has been loaded with zygotes, the three-way valve is configured to a "closed" position wherein fluid flow between the catheter assembly 8 and the extension hose 14 is closed and a fluid-flow connection between the extension hose 14 and the side vent port 117 is open. By sealing the proximal end of the catheter assembly 8, the vacuum associated with disconnecting the extension hose 14 from the port 20 on the control unit 12 will not displace the column of fluid (and zygotes) in the inner catheter 4. The catheter assembly 8 is then mounted in the socket on the upper portion of the barrel and in the nosepiece in the configuration shown in Figs. 12, 16 and 17.

With the catheter assembly 8 held snugly on the injector 160, the medical professional carries the "loaded" catheter to the implant situs, usually a hospital procedure room. At that location, the medical professional then reconnects the proximal end of the extension hose 14 to the port 20 of a second control unit 12. The three-way vent is then returned the open position. Because the three-way vent was configured in the closed position, the pressure created in the extension hose 14 during connection to the port 20 will not displace the column of fluid (and zygotes) in the inner catheter 4.

In accordance with a method of another preferred example, the sheath 6 of the catheter assembly is initially retracted relative to the distal tip 4b of the inner catheter 4. The medical professional then inserts the catheter assembly 8 through the cervix and into the uterus of the patient using one hand to hold the injector and the other hand to manipulate the catheter assembly 8. While watching the distal tip of the catheter assembly 8 on an imaging apparatus, the medical professional manipulates the distal tip 4b to the preferred implant site within the uterus. If the distal tip 4b becomes obstructed, the medical professional gradually extends the sheath by sequentially depressing and releasing the trigger 176 with a single hand, thereby stiffening the distal portion of the catheter assembly 8. With the sheath 6 extended, the catheter assembly 8 can more easily bypass the obstruction.

Once the distal tip 4b of the inner catheter 4 is precisely located at the desired implant site within the uterus, the medical professional dispenses zygotes from the catheter assembly 8 by activating the control unit 12, which has been preprogrammed with the desired fluid flow conditions including flow rate, pause time (if desired), turn off time, etc. Alternatively or additionally, the medical professional can control expulsion through the catheter assembly 8 using the foot switches 25 or other remote control means connected to the control unit. Using the above-described novel apparatus, a single medical professional can then easily pick up, transfer and implant numerous zygotes since fluid flow is controlled by the control unit 12. The professional can set the control unit 12 for a very slow or gentle volumetric fluid flow rate, a rapid flow rate, or something in between. A single professional can then use both hands to manipulate the catheter assembly 8 during the IVF procedure.

The foregoing is considered as illustrative only of the principles of the invention. Further, since numerous modifications and changes will readily occur to those skilled in the art, it is not desired to limit the invention to the exact construction and operation shown and described herein, and accordingly, all suitable modifications and equivalents may be resorted to, falling within the scope of the invention.

## Claims

1. An apparatus (10) for performing an *in vitro* fertilization procedure, said apparatus comprising;
a. a control unit (12) having an external port (20) and means for creating positive and negative fluid pressure at said port (20); and
b. a flexible extension hose (14) having a first coupling (34) at a proximal end (14a), which can be connected in sealed fluid communication to said port (20), and a second coupling (34) at the distal end (14b), which can be constructed and arranged to connect to a fitting (5) of a catheter assembly (8), further comprising;
c. an inter-uterine catheter assembly (8) connected to said second coupling (34), said catheter assembly (8) having:
a. an inner catheter (4) having a proximal end (4a), a quick connect/disconnect fitting (35) at the proximal end (4a) connectable to said second coupling, and an open distal end (4b); and,
b. an outer sheath (6) surrounding and slidable over said inner catheter (4) and having an open distal end (6b); **characterized in that** said extension hose (14) comprises;
a central tubular pneumatic passageway (28);
an inner sheath (32) surrounding and protecting said central tubular passageway (28); and
an outer sheath (30) surrounding said inner sheath (32) and protecting said central tubular passageway (28).

2. The apparatus (10) recited in claim 1, wherein said means for creating positive and negative fluid pressure at said port (20) comprise an internal pump (22) that is connected in pneumatic communication with said external port (20), said pump (22) being constructed and arranged to create either positive pressure or negative pressure at the port (20); and, said control unit (12) further comprises:
a. a housing (18); and
b. a command display (19) on the housing (18); and
c. a programmable microprocessor (24) that accepts input commands from said display (19) and controls operation of said pump (22).

3. The apparatus (10) recited in claim 2, further comprising a remote control means (25) for activating, de-activating, and reversing fluid flow generated by said pump (22).

4. The apparatus (10) recited in claim 1 wherein said control unit (12) can be programmed to repeatedly provide the same aspiration and expulsion flow conditions within the catheter assembly (8), and to provide a defined sequence of aspiration and expulsion fluid flow conditions within the catheter assembly (8).

5. The apparatus (10) recited in claim 1, wherein
said first and second couplings (34) of the extension hose (14) have a quick connect/disconnect construction;
each of said first and second couplings (34) comprises a Luer fitting (35) having a barbed connection stem, an insert (37) having a central axial aperture, and an adhesive affixing the central tubular passageway (28) in the central axial aperture of the insert (37), and
ends of the inner sheath (32), outer sheath (30) and central tubular passageway (28) are connected to the first and second couplings (34) such that pressurized air from the control unit (12) conveys only through the central tubular passageway (28).

6. The apparatus (10) recited in claim 5, wherein an outer surface of the central tubular passageway (28) is adhered inside the central axial aperture of said insert (37), the inner sheath (32) is compressed between the outer surface of the central tubular passageway (28) and an inner surface of the central axial aperture, and ends of the outer sheath (30) surround and connect to the barbed connection stem.

7. The apparatus (10) recited in claim 1, further comprising a carrier (60) constructed and arranged to support and temporarily lock the catheter assembly (8) in an orientation wherein the outer sheath (6) has been slid axially forward to cover a distal portion of the inner catheter (4), said carrier (60) comprising:
a. an elongate handle (62) having a longitudinal axis and opposed ends;
b. an elongate tray (64) fixed to one end of the elongate handle (62) and extending transversely to the longitudinal axis;
c. a distal socket (66) and a proximal socket (68) fixed at opposed ends of said elongate tray (64).

8. The apparatus (10) recited in claim 7, wherein at least a portion of an interior surface of each socket (66, 68) has a shape that compliments at least a portion of an exterior surface of the quick connect/disconnect fittings (35) of the catheter assembly (8), and at least a portion of the interior surface of the distal socket (66) has a shape that compliments at least a portion of the outer sheath (6).

9. The apparatus (10) recited in claim 1, further comprising an injector (160) constructed and arranged to support and temporarily lock the outer sheath (6) in a plurality of positions relative to the catheter (4), and to extend and retract the outer sheath (6) relative to the catheter (4), wherein said injector (160) can retract the outer sheath (6) proximally relative to the catheter assembly (8) to a first limit position exposing a distal tip of the inner catheter (4), and can extend the outer sheath (6) distally relative to the catheter assembly (8) to a second limit position covering the distal tip of the inner catheter (4).

10. The apparatus (10) recited in claim 9, wherein said injector (160) is constructed and arranged to be held and operated using a single hand.

11. The apparatus (10) recited in claim 10, wherein said injector (160) comprises:
a. a gun-shaped housing (162) including a handle (164), and a barrel (166) having a distal end (168) and a proximal end (170) relative to the handle (164);
b. a catheter assembly mount supported by said barrel (166);
c. a trigger (176) rotatably connected to an upper portion of the handle (164);
d. a sheath extender and retractor assembly (190) mounted in the barrel (166) and connected to said trigger (176).

12. The apparatus (10) recited in claim 11, wherein said sheath extender and retractor assembly (190) include:
a. an elongate activation rod (192) having a distal portion (192b) extending from the distal end (168) of said barrel (166) and a proximal portion (192a) slidably mounted in said barrel (166);
b. an advancement assembly connected to and activated by said trigger (176), which engages the proximal portion (192a) of said activation rod (192);
c. a nosepiece (196) fixed to the distal portion (192b) of said activation rod (192); and,
d. a retraction knob (218) fixed to the proximal end (192a) of said activation rod (192), and
wherein said catheter assembly mount comprises a proximal socket (68) formed in an upper surface of said barrel (166) and a distal socket (66) formed in said nosepiece (196);
said extension hose (14) including a three-way vent (116) arranged in fluid communication with said central tubular passageway (28) and fixed proximate said distal coupling (34); and,
a shape of said proximal socket (68) compliments a shape of said three-way vent (116) and a shape of said distal coupling (34) so that said vent (116) and distal coupling (34) can temporarily nest in said proximal socket (68), and wherein a shape of said distal socket (66) compliments a shape of the outer sheath (6) so that at least a portion of said outer sheath (6) can temporarily nest in said distal socket (66).

## Patentansprüche

1. Vorrichtung (10) zur Durchführung eines In-vitro-Fertilisationsverfahrens, wobei die Vorrichtung umfasst;
a. eine Steuereinheit (12) mit einem externen Anschluss (20) und Mitteln zur Erzeugung von positivem und negativem Fluiddruck an diesem Anschluss (20); und
b. einen flexiblen Verlängerungsschlauch (14) mit einer ersten Kupplung (34) an einem proximalen Ende (14a), die in abgedichteter Fluidverbindung mit dem Anschluss (20) verbunden werden kann, und einer zweiten Kupplung (34) an dem distalen Ende (14b), die so konstruiert und angeordnet sein kann, dass sie mit einem Fitting (5) einer Katheteranordnung (8) verbunden sein kann, ferner umfassend:
c. eine interuterine Katheteranordnung (8), die mit der zweiten Kupplung (34) verbunden ist, wobei die Katheteranordnung (8) aufweist:
a. einen inneren Katheter (4) mit einem proximalen Ende (4a), einem Schnellverbindungs-/Trennfitting (35) an dem proximalen Ende (4a), der mit der zweiten Kupplung verbindbar ist, und einem offenen distalen Ende (4b); und
b. einen äußeren Mantel (6), der den inneren Katheter (4) umgibt und darüber schiebbar ist und ein offenes distales Ende (6b) aufweist;
**dadurch gekennzeichnet, dass** der Verlängerungsschlauch (14) umfasst;
einen zentralen rohrförmigen pneumatischen Durchgang (28);
einen inneren Mantel (32), der den zentralen rohrförmigen Durchgang (28) umgibt und schützt; und
einen äußeren Mantel (30), der den inneren Mantel (32) umgibt und den zentralen rohrförmigen Durchgang (28) schützt.

2. Vorrichtung (10) nach Anspruch 1, wobei die Mittel zum Erzeugen von positivem und negativem Fluiddruck an dem Anschluss (20) eine interne Pumpe (22) umfassen, die in pneumatischer Verbindung mit dem externen Anschluss (20) verbunden ist, wobei die Pumpe (22) so konstruiert und angeordnet ist, dass sie entweder positiven Druck oder negativen Druck an dem Anschluss (20) erzeugt; und wobei die Steuereinheit (12) ferner umfasst:
a. ein Gehäuse (18); und
b. eine Befehlsanzeige (19) an dem Gehäuse (18); und
c. einen programmierbaren Mikroprozessor (24), der Eingabebefehle von der Anzeige (19) entgegennimmt und den Betrieb der Pumpe (22) steuert.

3. Vorrichtung (10) nach Anspruch 2, die ferner eine Fernsteuerungseinrichtung (25) zum Aktivieren, Deaktivieren und Umkehren der von der Pumpe (22) erzeugten Fluidströmung umfasst.

4. Vorrichtung (10) nach Anspruch 1, wobei die Steuereinheit (12) so programmiert sein kann, dass sie wiederholt die gleichen Ansaug- und Ausstoßströmungsbedingungen innerhalb der Katheteranordnung (8) bereitstellt und eine definierte Abfolge von Ansaug- und Ausstoßfluidströmungsbedingungen innerhalb der Katheteranordnung (8) bereitstellt.

5. Vorrichtung (10) nach Anspruch 1, wobei
die ersten und zweiten Kupplungen (34) des Verlängerungsschlauchs (14) eine Schnellkupplungs-/Trennkupplungskonstruktion aufweisen;
jede der ersten und zweiten Kupplungen (34) ein Luer-Fitting (35) mit einem mit Widerhaken versehenen Verbindungsschaft, einen Einsatz (37) mit einer zentralen axialen Öffnung und einen Klebstoff umfasst, der den zentralen röhrenförmigen Durchgang (28) in der zentralen axialen Öffnung des Einsatzes (37) fixiert, und
Enden des inneren Mantels (32), des äußeren Mantels (30) und des zentralen rohrförmigen Durchgangs (28) mit den ersten und zweiten Kupplungen (34) derart verbunden sind, dass Druckluft von der Steuereinheit (12) nur durch den zentralen rohrförmigen Durchgang (28) strömt.

6. Vorrichtung (10) nach Anspruch 5, wobei eine Außenfläche des zentralen rohrförmigen Durchgangs (28) in die zentrale axiale Öffnung des Einsatzes (37) geklebt ist, der innere Mantel (32) zwischen der Außenfläche des zentralen rohrförmigen Durchgangs (28) und einer Innenfläche der zentralen axialen Öffnung zusammengedrückt wird, und Enden des äußeren Mantels (30) den mit Widerhaken versehenen Verbindungsschaft umgeben und mit diesem verbunden sind.

7. Vorrichtung (10) nach Anspruch 1, die ferner einen Träger (60) umfasst, der so konstruiert und angeordnet ist, dass er die Katheteranordnung (8) in einer Ausrichtung hält und vorübergehend arretiert, wobei der äußere Mantel (6) axial nach vorne geschoben worden ist, um einen distalen Abschnitt des inneren Katheters (4) zu bedecken, wobei der Träger (60) umfasst:
a. einen länglichen Griff (62) mit einer Längsachse und gegenüberliegenden Enden;
b. eine längliche Schale (64), die an einem Ende des länglichen Griffs (62) befestigt ist und sich quer zur Längsachse erstreckt;
c. eine distale Aufnahme (66) und eine proximale Aufnahme (68), die an gegenüberliegenden Enden der länglichen Schale (64) befestigt sind.

8. Vorrichtung (10) nach Anspruch 7, wobei mindestens ein Abschnitt einer Innenfläche jeder Aufnahme (66, 68) eine Form hat, die zu mindestens einem Abschnitt einer Außenfläche der Schnellverbindungs-/Trennfittinge (35) der Katheteranordnung (8) passt, und mindestens ein Abschnitt der Innenfläche der distalen Aufnahme (66) eine Form hat, die zu mindestens einem Abschnitt des äußeren Mantels (6) passt.

9. Vorrichtung (10) nach Anspruch 1, die ferner einen Injektor (160) umfasst, der so konstruiert und angeordnet ist, dass er den äußeren Mantel (6) in einer Vielzahl von Positionen relativ zum Katheter (4) hält und vorübergehend arretiert, und dass er den äußeren Mantel (6) relativ zu dem Katheter (4) auszieht und zurückzieht, wobei der Injektor (160) den äußeren Mantel (6) proximal relativ zu der Katheteranordnung (8) in eine erste Grenzposition zurückziehen kann, die eine distale Spitze des inneren Katheters (4) freilegt, und den äußeren Mantel (6) distal relativ zu der Katheteranordnung (8) in eine zweite Grenzposition ausziehen kann, die die distale Spitze des inneren Katheters (4) bedeckt.

10. Vorrichtung (10) nach Anspruch 9, wobei der Injektor (160) so konstruiert und angeordnet ist, dass er mit einer einzigen Hand gehalten und bedient werden kann.

11. Vorrichtung (10) nach Anspruch 10, wobei der Injektor (160) umfasst:
a. ein pistolenförmiges Gehäuse (162), das einen Griff (164) und einen Lauf (166) mit einem distalen Ende (168) und einem proximalen Ende (170) in Bezug auf den Griff (164) enthält;
b. eine Katheteranordnungshalterung, die von dem Lauf (166) getragen ist;
c. einen Abzug (176), der drehbar mit einem oberen Abschnitt des Griffs (164) verbunden ist;
d. eine im Lauf (166) montierte und mit dem Abzug (176) verbundene Mantelauszugs- und -rückzugsanordnung (190).

12. Vorrichtung (10) nach Anspruch 11, wobei die Mantelauszugs- und -rückzugsanordnung (190) umfasst:
a. eine längliche Aktivierungsstange (192) mit einem distalen Abschnitt (192b), der sich von dem distalen Ende (168) des Laufs (166) erstreckt, und einem proximalen Abschnitt (192a), der verschiebbar in dem Lauf (166) angebracht ist;
b. eine Vorschubanordnung, die mit dem Abzug (176) verbunden ist und durch diesen aktiviert wird, und die mit dem proximalen Abschnitt (192a) der Aktivierungsstange (192) in Eingriff steht;
c. ein Nasenstück (196), das an dem distalen Abschnitt (192b) der Aktivierungsstange (192) befestigt ist; und
d. einen Rückzugsknopf (218), der an dem proximalen Ende (192a) der Aktivierungsstange (192) befestigt ist, und
wobei die Katheteranordnungshalterung eine proximale Aufnahme (68), die in einer oberen Fläche des Laufs (166) ausgebildet ist, und eine distale Aufnahme (66), die in dem Nasenstück (196) ausgebildet ist, umfasst;
der Verlängerungsschlauch (14) eine Drei-Wege-Entlüftung (116) aufweist, die in Fluidverbindung mit dem zentralen rohrförmigen Durchgang (28) angeordnet und in der Nähe der distalen Kupplung (34) befestigt ist; und
eine Form der proximalen Aufnahme (68) zu einer Form der Drei-Wege-Entlüftung (116) und einer Form der distalen Kupplung (34) passt, so dass die Entlüftung (116) und die distale Kupplung (34) vorübergehend in der proximalen Aufnahme (68) eingebettet sein können, und wobei eine Form der distalen Aufnahme (66) zu einer Form des äußeren Mantels (6) passt, so dass zumindest ein Abschnitt des äußeren Mantels (6) vorübergehend in der distalen Aufnahme (66) eingebettet sein kann.

## Revendications

1. Appareil (10) pour effectuer une procédure de fécondation in vitro, ledit appareil comprenant :
a. une unité de commande (12) ayant un orifice externe (20) et un moyen destiné à créer une pression fluidique positive et négative au niveau dudit orifice (20) ; et
b. un tuyau d'extension flexible (14) ayant un premier couplage (34) à une extrémité proximale (14a), qui peut être connecté en communication fluidique étanche audit orifice (20), et un second couplage (34) à l'extrémité distale (14b), qui peut être construit et agencé pour être connecté à un raccord (5) d'un assemblage de cathéter (8), comprenant en outre :
c. un assemblage de cathéter inter-utérin (8) connecté audit second couplage (34), ledit assemblage de cathéter (8) ayant :
a. un cathéter intérieur (4) ayant une extrémité proximale (4a), un raccord à connexion/déconnexion rapide (35) au niveau de l'extrémité proximale (4a) pouvant être connecté audit second couplage, et une extrémité distale ouverte (4b) ; et
b. une gaine extérieure (6) entourant ledit cathéter intérieur (4) et pouvant être glissée sur celui-ci, et ayant une extrémité distale ouverte (6b) ;
**caractérisé en ce que** ledit tuyau d'extension (14) comprend :
un passage pneumatique tubulaire central (28) ;
une gaine intérieure (32) entourant et protégeant ledit passage tubulaire central (28) ; et
une gaine extérieure (30) entourant ladite gaine intérieure (32) et protégeant ledit passage tubulaire central (28).

2. Appareil (10) selon la revendication 1, dans lequel ledit moyen destiné à créer une pression fluidique positive et négative au niveau dudit orifice (20) comprend une pompe interne (22) qui est connectée en communication pneumatique audit orifice externe (20), ladite pompe (22) étant construite et agencée pour créer soit une pression positive, soit une pression négative, au niveau de l'orifice (20) ; et ladite unité de commande (12) comprend en outre :
a. un boîtier (18) ; et
b. un affichage d'ordres (19) sur le boîtier (18) ; et
c. un microprocesseur programmable (24) qui accepte des ordres entrés depuis ledit affichage (19) et qui commande un fonctionnement de ladite pompe (22).

3. Appareil (10) selon la revendication 2, comprenant en outre un moyen de commande à distance (25) destiné à activer, à désactiver et à inverser un écoulement fluidique généré par ladite pompe (22).

4. Appareil (10) selon la revendication 1, dans lequel ladite unité de commande (12) peut être programmée pour fournir de manière répétée les mêmes conditions d'écoulement d'aspiration et d'expulsion à l'intérieur de l'assemblage de cathéter (8), et pour fournir une séquence définie d'écoulement fluidique d'aspiration et d'expulsion à l'intérieur de l'assemblage de cathéter (8).

5. Appareil (10) selon la revendication 1, dans lequel
ledit premier et ledit second couplage (34) du tuyau d'extension (14) ont une construction à connexion/déconnexion rapide ;
chacun dudit premier et dudit second couplage (34) comprend un raccord Luer (35) ayant une tige de connexion à barbillons, un insert (37) ayant une ouverture axiale centrale, et un adhésif fixant le passage tubulaire central (28) dans l'ouverture axiale centrale de l'insert (37), et
des extrémités de la gaine intérieure (32), de la gaine extérieure (30) et du passage tubulaire central (28) sont connectées au premier et au second couplage (34) de sorte que de l'air pressurisé provenant de l'unité de commande (12) est acheminé uniquement à travers le passage tubulaire central (28).

6. Appareil (10) selon la revendication 5, dans lequel une surface extérieure du passage tubulaire central (28) est collée à l'intérieur de l'ouverture axiale centrale dudit insert (37), la gaine intérieure (32) est comprimée entre la surface extérieure du passage tubulaire central (28) et une surface intérieure de l'ouverture axiale centrale, et des extrémités de la gaine extérieure (30) entourent et sont connectées à la tige de connexion à barbillons.

7. Appareil (10) selon la revendication 1, comprenant en outre un moyen de support (60) construit et agencé pour supporter et verrouiller temporairement l'assemblage de cathéter (8) dans une orientation, dans lequel la gaine extérieure (6) a été glissée axialement vers l'avant pour couvrir une portion distale du cathéter intérieur (4), ledit moyen de support (60) comprenant :
a. une poignée allongée (62) ayant un axe longitudinal et des extrémités opposées ;
b. un plateau allongé (64) fixé à une extrémité de la poignée allongée (62) et s'étendant transversalement à l'axe longitudinal ;
c. un manchon distal (66) et un manchon proximal (68) fixés à des extrémités opposées dudit plateau allongé (64).

8. Appareil (10) selon la revendication 7, dans lequel au moins une portion d'une surface intérieure de chaque manchon (66, 68) a une forme qui correspond à au moins une portion d'une surface extérieure des raccords à connexion/déconnexion rapide (35) de l'assemblage de cathéter (8), et au moins une portion de la surface intérieure du manchon distal (66) a une forme qui correspond à au moins une portion de la gaine extérieure (6).

9. Appareil (10) selon la revendication 1, comprenant en outre un injecteur (160) construit et agencé pour supporter et verrouiller temporairement la gaine extérieure (6) dans une pluralité de positions relativement au cathéter (4), et pour élargir et rétracter la gaine extérieure (6) relativement au cathéter (4), dans lequel ledit injecteur (160) peut rétracter la gaine extérieure (6) de manière proximale relativement à l'assemblage de cathéter (8) jusqu'à une première position limite exposant une pointe distale du cathéter intérieur (4), et peut élargir la gaine extérieure (6) de manière distale relativement à l'assemblage de cathéter (8) jusqu'à une seconde position limite couvrant la pointe distale du cathéter intérieur (4).

10. Appareil (10) selon la revendication 9, dans lequel ledit injecteur (160) est construit et agencé pour être maintenu et actionné en utilisant une seule main.

11. Appareil (10) selon la revendication 10, dans lequel ledit injecteur (160) comprend :
a. un boîtier en forme de pistolet (162) incluant une poignée (164), et un cylindre (166) ayant une extrémité distale (168) et une extrémité proximale (170) relativement à la poignée (164) ;
b. une monture d'assemblage de cathéter supportée par ledit cylindre (166) ;
c. une gâchette (176) connectée en rotation à une portion supérieure de la poignée (164) ;
d. un assemblage d'extension et de rétraction de gaine (190) monté dans le cylindre (166) et connecté à ladite gâchette (176).

12. Appareil (10) selon la revendication 11, dans lequel ledit assemblage d'extension et de rétraction de gaine (190) inclut :
a. une barre d'activation allongée (192) ayant une portion distale (192b) s'étendant depuis l'extrémité distale (168) dudit cylindre (166) et une portion proximale (192a) montée de manière coulissante dans ledit cylindre (166) ;
b. un assemblage d'avancement connecté à et activé par ladite gâchette (176), qui engage la portion proximale (192a) de ladite barre d'activation (192) ;
c. un embout (196) fixé à la portion distale (192b) de ladite barre d'activation (192) ; et
d. un bouton de rétraction (218) fixé à l'extrémité proximale (192a) de ladite barre d'activation (192), et
dans lequel ladite monture d'assemblage de cathéter comprend un manchon proximal (68) formé dans une surface supérieure dudit cylindre (166) et un manchon distal (66) formé dans ledit embout (196) ;
ledit tuyau d'extension (14) incluant un évent à trois voies (116) agencé en communication fluidique avec ledit passage tubulaire central (28) et fixé à proximité dudit couplage distal (34) ; et
une forme dudit manchon proximal (68) correspond à une forme dudit évent à trois voies (116) et à une forme dudit couplage distal (34) de sorte que ledit évent (116) et ledit couplage distal (34) peuvent nicher temporairement dans ledit manchon proximal (68), et dans lequel une forme dudit manchon distal (66) correspond à une forme de la gaine extérieure (6) de sorte qu'au moins une portion de ladite gaine extérieure (6) peut temporairement nicher dans ledit manchon distal (66).
